## Europäisches Patentamt

## European Patent Office

## Office européen des brevets

(19)

(11) Veröffentlichungsnummer: **0 082 316**
**B1**

(12) **EUROPÄISCHE PATENTSCHRIFT**

(45) Veröffentlichungstag der Patentschrift:
**03.07.85**

(51) Int. Cl.⁴: **C 07 C 43/04**, C 07 C 41/06,
C 07 C 7/148

(21) Anmeldenummer: **82110734.9**

(22) Anmeldetag: **20.11.82**

(54) **Verfahren zur Herstellung von Methyl-Tert.-butylether (MTBE) und weitgehend von i-Buten und von Methanol befreiten Kohlenwasserstoff-Raffinaten und Vorrichtung hierfür.**

(30) Priorität: **04.12.81 DE 3148109**

(43) Veröffentlichungstag der Anmeldung:
**29.06.83 Patentblatt 83/26**

(45) Bekanntmachung des Hinweises auf die Patenterteilung:
**03.07.85 Patentblatt 85/27**

(84) Benannte Vertragsstaaten:
**BE DE FR GB IT**

(56) Entgegenhaltungen:
**DE - A - 2 802 198**
**DE - A - 2 802 199**
**DE - A - 2 853 769**
**DE - A - 2 908 426**

(73) Patentinhaber: **EC ERDÖLCHEMIE GMBH,
Postfach 75 20 02, D-5000 Köln 71 (DE)**

(72) Erfinder: **Schleppinghoff, Bernhard, Dr., Adolf Kolping
Strasse 5, D-4047 Dormagen 1 (DE)**
Erfinder: **Becker, Martin, Dipl.-Ing., Am Frohnweiher 9,
D-5000 Köln 71 (DE)**

(74) Vertreter: **Mann, Volker, Dr. et al, c/o Bayer
Aktiengesellschaft Zentralbereich Patente Marken und
Lizenzen, D-5090 Leverkusen-Bayerwerk (DE)**

## Beschreibung

Die vorliegende Erfindung betrifft ein Verfahren zu gleichzeitigen Herstellung von Methyl-tert.-butylether (MTBE) und von Kohlenwasserstoff-Raffinaten, wobei beide weitgehend von i-Buten und von Methanol frei sind, sowie eine hierfür geeignete Vorrichtung.

MTBE dient beispielsweise als Vergaserkraftstoff-Additiv zur Octanzahlverbesserung und als Löse- und Extraktionsmittel, das im Gegensatz zu vielen anderen Ethern fast keine Peroxide bildet. Ausserdem eignet sich MTBE als Ausgangsstoff zur Darstellung von reinem i-Buten über die Etherspaltung.

Es ist bekannt, i-Buten und Methanol an sauren Ionenaustauschern zu MTBE umzusetzen (DE-PS Nr. 1224294). Da diese Reaktion sehr selektiv ist, eignen sich als Einsatzstoffe besonders auch Gemische von i-Buten mit anderen Kohlenwasserstoffen, wie sie beispielsweise in thermischen oder katalytischen Crackern anfallen.

Es wird angestrebt, das i-Buten in solchen Gemischen möglichst weitgehend umzusetzen. Hierzu wird im allgemeinen ein grosser Methanolüberschuss eingesetzt (DE-OS Nr. 2853769). Ein möglichst weitgehender i-Buten-Umsatz in den $C_4$-Raffinaten wird auch aus dem Grunde angestrebt, weil zur Weiterverwertung des i-Buten-freien $C_4$-Raffinats der Restgehalt an i-Buten unter 2 Gew.-%, nach Möglichkeit sogar unter 1 oder 0,5 Gew.-% liegen soll. Diese Spezifikation ist beispielsweise erforderlich, wenn Maleinsäureanhydrid, Methyl-ethylketon, Buten-1 oder Octene (durch Dimerisierung) aus diesen meist als $C_4$-Raffinat II bezeichneten Restgemischen hergestellt werden soll. Unter diesem Gesichtspunkt der Weiterverarbeitung muss ausserdem dafür gesorgt werden, dass im $C_4$-Raffinat II kein Methanol mehr vorliegt. Insofern werden an das Erreichen eines spezifikationsgerechten Raffinats besondere Anforderungen gestellt. Aufgrund azeotroper Effekte hat sich jedoch gezeigt, dass durch einfache Destillation ein methanolfreies $C_4$-Raffinat II nicht erhalten werden kann (vgl. DE-OS Nr. 2802198, Seite 3, Absatz 3).

Der Methanolgehalt im MTBE sollte aus Gründen der Weiterverarbeitung, des Transportes und der Lagerung 3 Gew.-% bis 5 Gew.-% nicht überschreiten. Da auch MTBE mit nicht umgesetztem Methanol ein Azeotrop bildet, ist eine Trennung durch einfache Destillation nicht möglich. Zur Entfernung des Methanols aus dem $C_4$-Raffinat II und aus dem MTBE/Methanol-Azeotrop wurde als brauchbare Methode eine Wasserwäsche vorgeschlagen (DE-OS Nr. 2246004, US Nr. 3726942). Die Aufarbeitung des dabei entstehenden wässerigen Methanols und die Trocknung der zurückbleibenden Stoffe, nämlich MTBE und $C_4$-Raffinat II stellen jedoch unerwünschte zusätzliche Schritte mit beträchtlichen Betriebskosten und hohen Investitionen dar.

Eine weitere Verfahrensvariante zur Erzielung hoher i-Buten-Umsätze ist die zweistufige Veretherung mit einer zwischengeschalteten $C_4$-

Abtrennung (DE-OS Nr. 2521964, US Nr. 3979461). Das Reaktionsprodukt der ersten Stufe wird in einer Destillationskolonne aufgetrennt. Das Kopfprodukt, das noch unumgesetztes i-Buten und Methanol enthält, wird in einer zweiten Stufe einer Nachreaktion zugeführt. Das Reaktionsprodukt der zweiten Stufe wird in einer zweiten Destillationskolonne aufgetrennt. Dieses Verfahren erfordert ebenfalls hohen Energie- und Investitionsaufwand.

Es wurde nun ein Verfahren zur Herstellung von Methyl-tert.-butylether (MTBE) und weitgehend von i-Buten und von Methanol freiem Kohlenwasserstoff-Raffinat aus Methanol und einem i-Buten enthaltenden Kohlenwasserstoffgemisch durch Veretherung an sauren Kationenaustauschern gefunden, das dadurch gezeichnet ist, dass man Methanol und das im Kohlenwasserstoffgemisch enthaltene i-Buten im molaren Verhältnis von 0,9:1 bis 1:0,9 einsetzt, bei der destillativen Auftrennung des Veretherungsgemisches in seine Bestandteile einen Seitenstrom aus der Destillationskolonne entnimmt und diesen nochmals einer Veretherung unterwirft.

Als i-Buten enthaltendes Kohlenwasserstoffgemisch sei beispielsweise ein $C_4$-Schnitt, wie er beim thermischen Cracken nach der Entfernung des Butadiens und gegebenenfalls der Aromaten anfällt, oder ein $C_4$-Schnitt aus einer katalytischen Crackanlage genannt. Die typische Zusammensetzung solcher $C_4$-Schnitte ist in der folgenden Tabelle aufgeführt:

*Tabelle*

|  | $C_4$ aus Steam-Cracker | $C_4$ aus Cat-Cracker |
|---|---|---|
| n-Butan | 6- 8 Gew.-% | etwa 10 Gew.-% |
| i-Butan | 2- 3 Gew.-% | etwa 34 Gew.-% |
| i-Buten | 40-45 Gew.-% | etwa 15 Gew.-% |
| n-Buten-1 | 24-28 Gew.-% | etwa 13 Gew.-% |
| n-Buten-2 | 19-21 Gew.-% | etwa 28 Gew.-% |

Das Methanol wird dem i-Buten-haltigen Einsatz-Kohlenwasserstoffgemisch im wesentlichen in äquimolarer Menge, bezogen auf das im Kohlenwasserstoffgemisch enthaltene i-Buten, eingesetzt, also im molaren Verhältnis von Methanol zu i-Buten wie 0,9 zu 1 bis 1 zu 0,9, bevorzugt 0,95 zu 1 bis 1 zu 0,95, besonders bevorzugt etwa 1 zu 1.

Die Veretherungsreaktion erfolgt in bekannter Weise an einem sauren Kationenaustaucher, beispielsweise einem Sulfonsäuregruppen-haltigen Styrol-divinylbenzol-Polymerisat in fester oder schwebender Schicht bei einer Temperatur von 30 bis 120°C, bevorzugt 40 bis 90°C, und einem Druck von 1 bis 50 bar, bevorzugt 3 bis 20 bar mit einer stündlichen Kontaktbelastung (WHSV = weight hourly space velocity) von 0,1 bis 15 kg Gesamteinsatzstoffe pro Kilogramm Kationenaustauscher pro Stunde. Die genannten Temperaturen und Drucke werden hierbei so zueinander eingestellt, dass die Veretherungsreaktion in flüssiger Phase abläuft.

Das den Veretherungsreaktor verlassende Veretherungsgemisch kann beispielsweise zur besseren Steuerung der Reaktionstemperatur aufgeteilt werden, wobei ein Teilstrom über einen Kühler und eine Umwälzpumpe wieder an den Eingang des Veretherungsreaktors zurückgeführt und gemeinsam mit frischem Einsatzmaterial den Veretherungsreaktor noch einmal durchfährt. Der andere Teil des aufgeteilten Veretherungsgemisches kann sodann der Aufarbeitung zugeführt werden. In einer weiteren Variante kann jedoch auch dieser zweite Teil des aufgeteilten Veretherungsgemisches in einen zweiten Veretherungsreaktor gefahren werden und erst nach Durchlaufen dieses zweiten Veretherungsreaktors der Aufarbeitung zugeführt werden. Diese zweite Verfahrensvariante trägt ebenfalls zur Erhöhung des Umsatzes bei.

Nach Durchlaufen der gesamten Veretherungsstufe wird das Veretherungsgemisch durch Destillation aufgetrennt und erfindungsgemäss ein Teil des Veretherungsgemisches nochmals einer Veretherungsreaktion unterworfen. Hierzu wird das Veretherungsgemisch in eine Destillationskolonne eingespeist. Diese Kolonne hat beispielsweise 30 bis 80, bevorzugt 40 bis 70 Böden. Sie kann als Glockenbodenkolonne oder als Siebbodenkolonne ausgeführt sein. Diese Destillationskolonne wird bei einem Druck von 1 bis 10 bar, bevorzugt 3 bis 7 bar, besonders bevorzugt 4 bis 6 bar betrieben.

Oberhalb der Einspeisungsstelle für das Veretherungsgemisch zur Destillationskolonne wird ein flüssiger Seitenstrom entnommen. Die Menge dieses Seitenstroms liegt etwa bei 50 bis 200%, bevorzugt 75 bis 150% der Menge des am Kopf der Destillationskolonne entnommenen weitgehend vom i-Buten und vom Methanol freien Kohlenwasserstoff-Raffinats. Dieser abgezogene Seitenstrom wird in einen mit saurem Kationenaustauscher gefüllten Nachreaktor geführt, der beispielsweise als Festbettreaktor ausgelegt ist. Als saurer Kationenaustauscher kann der gleiche eingesetzt werden, der in der Veretherungsreaktion, die oben beschrieben worden ist, eingesetzt wird. Beispielsweise sei auch hier ein Sulfonsäuregruppenhaltiger Styrol-Divinylbenzol-Austauscher in der $H^+$-Form genannt. Jedoch eignen sich auch alle anderen stark sauren Kationenaustauscher in der $H^+$-Form, etwa auf Phenol-Formaldehyd-Basis, wie sie dem Fachmann bekannt sind. Als Kontaktbelastung durch den entnommenen Destillatstrom kann die gleiche WHSV genommen werden, die oben für die Veretherungsreaktion genannt wurde. Bevorzugt wird eine WHSV von 1 bis 5 eingestellt.

Am Eingang dieses Nachreaktors hat der entnommene Seitenstrom beispielsweise eine Temperatur von 30 bis 100°C, bevorzugt 40 bis 70°C. Nach dem Durchlaufen dieses Nachreaktors wird der entnommene Seitenstrom unterhalb seiner Entnahmestelle in den Prozess zurückgeführt. Diese Rückführung in den Prozess kann beispielsweise durch Einspeisung in die Ausgangsstoffe Methanol und $C_4$-Raffinat I vor Eintritt in den Veretherungsreaktor, durch Einspeisung vor Eintritt in einen zweiten Veretherungsreaktor oder durch Einspeisung in die Destillationskolonne durchgeführt werden. Die Rückführung in die Destillationskolonne kann in den Abtriebsteil oder in den Verstärkerteil bis unterhalb der Entnahmestelle des Seitenstromes erfolgen, beispielsweise gemeinsam mit der Einspeisung des vom Veretherungsreaktor ablaufenden Reaktionsgemisches in die Destillationskolonne. Diese Rückführung in die Destillationskolonne ist bevorzugt. Besonders bevorzugt ist die Rückführung in den Verstärkerteil der Destillationskolonne von der Einspeisung des Reaktionsgemisches bis unterhalb der Entnahmestelle des Seitenstromes für den Nachreaktor.

Der Nachreaktor wird bei einem Druck betrieben, der entsprechend der Eingangstemperatur ausreicht, um den entnommenen Seitenstrom während des Durchlaufs durch diesen Nachreaktor in der flüssigen Phase zu halten.

In besonders bevorzugter Weise wird der entnommene Seitenstrom bei der Temperatur in den Nachreaktor geführt, die an der Entnahmestelle der Destillationskolonne herrscht.

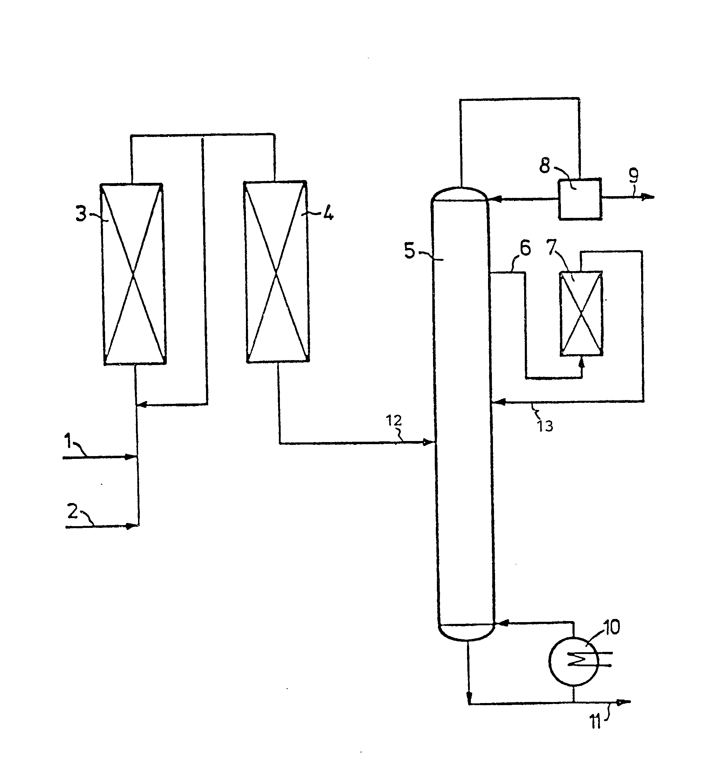

Die allgemeine Durchführung des erfindungsgemässen Verfahrens sei beispielhaft anhand der beiliegenden Figur beschrieben:

Das i-Buten-haltige $C_4$-Gemisch 1 und eine dem Gehalt an i-Buten etwa äquivalente Menge Methanol 2 werden in den Reaktor 3 eingebracht. Dieser Reaktor kann beispielsweise ein Festbettreaktor oder ein Röhrenreaktor sein.

Der Ausgangsstrom des Reaktors kann beispielsweise aufgeteilt werden, wobei ein Teil wieder mit dem Eingangsstrom für den Reaktor 3 vereinigt wird, während der andere Teilstrom in einen zweiten Reaktor 4 geführt wird, der im allgemeinen mit dem gleichen Katalysator wie 3 gefüllt ist. Der Katalysator im zweiten Reaktor 4 kann im allgemeinen als Festbettkatalysator angeordnet sein. Das flüssige Ausgangsprodukt des zweiten Reaktors 4 wird in eine Destillationskolonne 5 eingebracht. Oberhalb der Einspeisungsstelle 12 des Veretherungsgemisches zur Destillationskolonne wird der Destillationskolonne flüssig ein Seitenstrom 6 entnommen und beispielsweise von unten nach oben durch einen Nachreaktor 7 geführt. Der Katalysator in diesem Nachreaktor 7 kann beispielsweise der gleiche sein wie in den zuvor beschriebenen Reaktoren 3 und 4 und beispielsweise als Festbett angeordnet sein. Nach Verlassen des Nachreaktors 7 gelangt der entnommene Seitenstrom unterhalb seiner Entnahmestelle in die Destillationskolonne zurück. Eine Temperaturregelung ist im allgemeinen nicht notwendig. Das Kopfprodukt der Destillationskolonne 5 wird in einem Kühler kondensiert und aus einem Kondensatteiler 8 teilweise als Rückfluss auf den Kopf der Kolonne zurückgegeben. Das weitgehend i-Buten-freie Kopfprodukt 9 wird aus dem Kondensatteiler 8 entnommen. Das Rückflussverhältnis (Rückfluss/Entnahme = R/E) wird im allgemeinen auf einen Wert von 0,1 bis 5, bevorzugt von 0,5 bis 2 eingestellt. Das Sumpfprodukt der Kolonne wird zum Teil über den Umlauferhitzer 10 umgepumpt, zum Teil wird es als reine MTBE 11 entnommen.

Die Erfindung betrifft weiterhin eine Vorrichtung zur simultanen destillativen Aufarbeitung und nochmaligen Veretherung von Methyl-tert.-butylether (MTBE), i-Buten und Methanol enthaltenden Veretherungsgemischen, die durch eine Destillationskolonne 5 gekennzeichnet ist, die folgende Einrichtungen aufweist:

a) eine Einspeisungsstelle 12 für das Veretherungsgemisch,

b) am Sumpf einen an sich bekannten Umlauferhitzer 10 und eine Entnahmeeinrichtung 11 für das MTBE,

c) am Kopf einen an sich bekannten Kondensatteiler 8 für Rückfluss und Entnahme 9 des restlichen $C_4$-Gemisches, das frei von MTBE und weitgehend frei von i-Buten und Methanol ist,

d) eine Entnahmeleitung 6 für einen Seitenstrom oberhalb der Einspeisungsstelle 12 des Veretherungsgemisches in die Destillationskolonne 5, und

e) einen mit einem sauren Kationenaustauscher gefüllten Nachreaktor 7, in den der entnommene Seitenstrom geführt wird.

Die Rückführung des aus dem Nachreaktor 7 für die nochmalige Veretherung austretenden Gemisches kann in der oben beschriebenen Form in den Veretherungsreaktor 3, gegebenenfalls in einen zweiten Veretherungsreaktor 4 oder in die Destillationskolonne 5 erfolgen. Die erfindungsgemässe Vorrichtung weist daher in bevorzugter Form noch einen Zulauf 13 für das aus dem Nachreaktor 7 ablaufende Gemisch auf. In besonders bevorzugter Form befindet sich dieser Zulauf im Verstärkerteil der Destillationskolonne 5 von der Einspeisungsstelle 12 des Veretherungsgemisches bis unterhalb der Entnahmeleitung 6 für den Seitenstrom.

Mit Hilfe des erfindungsgemässen Verfahrens gelingt es, einen Kopfstrom in der als Debutanizer wirkenden Destillationskolonne 5 zu erhalten, der einen i-Buten-Gehalt von unter 0,6 Gew.-%, vielfach von unter 0,4 Gew.-% und einen Methanolgehalt von unter 0,8 Gew.-%, vielfach unter 0,6 Gew.-% besitzt. Ein solches $C_4$-Raffinat II eignet sich aufgrund der angegebenen geringen Beimengungen von i-Buten und Methanol im allgemeinen ohne weitere Behandlung zur Weiterverarbeitung, beispielsweise zur Herstellung von Maleinsäureanhydrid, Methyl-ethylketon, reinem Buten-1 sowie den Dimeren und Oligomeren des n-Butens.

Nur in wenigen Fällen wird es notwendig sein, auch den genannten kleinen Restgehalt an Methanol zu entfernen, beispielsweise ist dies vorteilhaft zur Buten-1-Gewinnung. Das Sumpfprodukt im erfindungsgemässen Verfahren ist ein weitgehend von Methanol freies MTBE, das direkt einer weiteren Verwendung, beispielsweise als Treibstoffzusatz oder Lösungsmittel, zugeführt werden kann.

Im Gegensatz zu Verfahren des Standes der Technik, die zur weitgehend vollständigen Umsetzung des i-Butens hohe Methanolüberschüsse mit entsprechend langen Verweilzeiten im Reaktionsteil oder eine mehrstufige Auftrennung der $C_4$-Fraktion mit entsprechend hohem Energieaufwand erfordern, erlaubt das erfindungsgemässe Verfahren kürzere Verweilzeiten, einen geringeren Energieverbrauch, geringere Investitionskosten und ermöglicht einen weitgehend vollständigen Methanol- und i-Buten-Umsatz ohne aufwendigere Verfahrensschritte. Als i-Buten-Umsatz sei beispielsweise ein solcher von über 99 Gew.-% genannt.

*Beispiel 1:*

500 ml (= 300 g) pro Stunde eines $C_4$-Gemisches mit 41 Gew.-% i-Buten (= 2,20 Mol) gelangt nach Vermischung mit 90 ml (= 71,9 g = 2,22 Mol) pro Stunde Methanol und Vorwärmung auf 50°C in einen Festbettreaktor von 0,75 m Länge und 2,5 cm Innendurchmesser, der mit 0,36 l eines sauren Kationenaustauschers auf der Basis eines Sulfonsäuregruppen-haltigen Styrol-Divinylbenzol-Harzes in makroporöser Form mit einer Ionenaustauschkapazität von 1,4 Mol/l gefüllt ist. Das Reaktionsprodukt dieses Reaktors wird teilweise im Kreis zurückgeführt, wobei es über einen Kühler auf die Reaktoreingangstemperatur gekühlt wird. Der Reaktor ist isoliert und arbeitet als adiabatischer Reaktor. Die Reaktorausgangstemperatur beträgt 77°C. Das Verhältnis von Frisch-$C_4$-Gemisch zu Kreislauf-Gemisch beträgt 1 zu 2. Das nicht zurückgeführte Reaktionsgemisch des ersten Reaktors gelangt nach Abkühlung auf 50°C in einen zweiten Festbettreaktor, der mit 0,75 l desselben Kontaktes gefüllt ist wie der erste Reaktor und eine Höhe von 1,6 m und einen Innendurchmesser von 2,5 cm hat. Das Reaktionsprodukt (Veretherungsgemisch) dieses zweiten Reaktors gelangt nach Entspannung auf 5 bar in eine Destillationskolonne mit 60 praktischen Böden. Der Zulauf ist am 15. Boden der Kolonne angebracht. Die Kolonne arbeitet bei einem Druck von 5 bar. Die Kopftemperatur der Kolonne beträgt 44°C, die Sumpftemperatur 107°C.

Am 45. Boden der Kolonne wird eine Menge von 250 ml pro Stunde flüssig entnommen und über eine Pumpe von unten in einen im Seitenstrom angeordneten Nachreaktor geführt. Der im Seitenstrom geschaltete Nachreaktor hat eine Höhe von 0,3 m und einen Durchmesser von 2,5 cm. Er ist mit dem oben beschriebenen sauren Kationenaustauscher gefüllt. Die Eingangstemperatur beträgt entsprechend der Entnahmestelle etwa 50°C. Das vom Nachreaktor abfliessende Gemisch gelangt am 30. Boden zurück in die Destillationskolonne.

Am Eingang des Nachreaktors beträgt der Gehalt an i-Buten 1,2 Gew.-% und der Methanolgehalt 1,0 Gew.-%. Am Ausgang des Nachreaktors ist der i-Buten-Gehalt auf 0,1 Gew.-% und der Methanolgehalt auf 0,3 Gew.-% zurückgegangen.

Das am Kopf entnommene $C_4$-Raffinat II hat folgende Zusammensetzung:

| | |
|---|---|
| n-Butan | 18,1 Gew.-% |
| i-Butan | 6,4 Gew.-% |
| i-Buten | 0,6 Gew.-% |
| n-Butan-1 | 43,9 Gew.-% |
| trans-Buten-2 | 18,3 Gew.-% |

cis-Buten-2    11,9 Gew.-%
Methanol    0,8 Gew.-%

Das entnommene Sumpfprodukt hat folgende Zusammensetzung:

| | |
|---|---|
| MTBE | 98,4 Gew.-% |
| Methanol | 0,3 Gew.-% |
| $C_4$-Kohlenwasserstoffe | 0,1 Gew.-% |
| $C_8$-Kohlenwasserstoffe | 0,9 Gew.-% |
| $H_2O$ | 0,2 Gew.-% |
| tert.-Butanol | 0,1 Gew.-% |

*Beispiel 2:*

500 ml (= 300 g) pro Stunde eines $C_4$-Gemisches mit 41 Gew.-% i-Buten (= 2,20 Mol) gelangt nach Vermischung mit 90 ml (= 71,9 g = 2,22 Mol) pro Stunde Methanol und Vorwärmung auf 50°C in einen Festbettreaktor von 0,75 m Länge und 2,5 cm Innendurchmesser, der mit 0,36 l eines sauren Kationenaustauschers auf der Basis eines Sulfonsäuregruppen-haltigen Styrol-Divinylbenzol-Harzes in makroporöser Form mit einer Ionenaustauschkapazität von 1,4 Mol/l gefüllt ist. Das Reaktionsprodukt dieses Reaktors wird teilweise im Kreis zurückgeführt, wobei es über einen Kühler auf die Reaktoreingangstemperaturen gekühlt wird. Der Reaktor ist isoliert und arbeitet als adiabatischer Reaktor. Die Reaktorausgangstemperatur beträgt 77°C. Das Verhältnis von Frisch-$C_4$-Gemisch zu Kreislauf-Gemisch beträgt 1 zu 2. Das nicht zurückgeführte Reaktionsgemisch des ersten Reaktors gelangt nach Abkühlung auf 50°C in einen zweiten Festbettreaktor, der mit 0,75 l desselben Kontaktes gefüllt ist wie der erste Reaktor und eine Höhe von 1,6 m und einen Innendurchmesser von 2,5 cm hat. Das Reaktionsprodukt (Veretherungsgemisch) dieses zweiten Reaktors gelangt nach Entspannung auf 5 bar in eine Destillationskolonne mit 60 praktischen Böden. Der Zulauf ist am 15. Boden der Kolonne angebracht. Die Kolonne arbeitet bei einem Druck von 5 bar. Die Kopftemperatur der Kolonne beträgt 44°C; die Sumpftemperatur 107°C. Der Kopfstrom der Kolonne wird in einem Kondensator verflüssigt und gelangt in einen Kondensatteiler. Aus dem Kondensatteiler werden zum einen 250 ml als Kopfprodukt entnommen, zum anderen 500 ml als Rücklauf über eine Pumpe von unten in einen Nachreaktor geführt. Der im Rücklauf geschaltete Nachreaktor hat eine Höhe von 0,3 m und einen Durchmesser von 2,5 cm. Er ist mit dem oben beschriebenen sauren Kationenaustauscher gefüllt. Die Temperatur im Reaktor beträgt etwa 45°C. Das vom Nachreaktor abfliessende Gemisch gelangt am obersten Boden zurück in die Destillationskolonne.

Am Eingang des Nachreaktors beträgt der Gehalt an i-Buten 0,4 Gew.-% und der Methanolgehalt 0,6 Gew.-%. Am Ausgang des Nachreaktors ist der i-Buten-Gehalt auf 0,1 Gew.-% und der Methanolgehalt auf 0,4 Gew.-% zurückgegangen.

Das am Kopf entnommene $C_4$-Raffinat II hat folgende Zusammensetzung:

| | |
|---|---|
| n-Butan | 18,0 Gew.-% |
| i-Butan | 6,4 Gew.-% |
| i-Buten | 0,4 Gew.-% |
| n-Butan-1 | 44,1 Gew.-% |
| trans-Buten-2 | 18,5 Gew.-% |
| cis-Buten-2 | 12,0 Gew.-% |
| Methanol | 0,6 Gew.-% |

Im Kopfprodukt kann kein MTBE nachgewiesen werden. Das entnommene Sumpfprodukt hat folgende Zusammensetzung:

| | |
|---|---|
| MTBE | 98,8 Gew.-% |
| Methanol | < 0,2 Gew.-% |
| $C_4$-Kohlenwasserstoffe | < 0,1 Gew.-% |
| $C_8$-Kohlenwasserstoffe | 0,9 Gew.-% |
| $H_2O$ | < 0,2 Gew.-% |
| tert.-Butanol | < 0,1 Gew.-% |

## Patentansprüche

1. Verfahren zur Herstellung von Methyl-tert.-butylether (MTBE) und weitgehend von i-Buten und von Methanol freiem Kohlenwasserstoff-Raffinat aus Methanol und einem i-Buten enthaltenden Kohlenwasserstoff-Gemisch durch Veretherung an sauren Kationenaustauschern, dadurch gekennzeichnet, dass man Methanol und das im Kohlenwasserstoff-Gemisch enthaltene i-Buten im molaren Verhältnis von 0,9:1 bis 1:0,9 einsetzt bei der destillativen Auftrennung des Veretherungsgemisches in seine Bestandteile einen Seitenstrom aus der Destillationskolonne entnimmt und diesen nochmals einer Veretherungsreaktion unterwirft.

2. Verfahren nach Anspruch 1, dadurch gekennzeichnet, dass für die nochmalige Veretherung ein Seitenstrom oberhalb der Einspeisungsstelle der Destillationskolonne für das Veretherungsgemisch entnommen und einem Nachreaktor zugeführt wird.

3. Verfahren nach den Ansprüchen 1 und 2, dadurch gekennzeichnet, das der Seitenstrom in flüssiger Phase der nochmaligen Veretherung zugeführt wird.

4. Verfahren nach den Ansprüchen 1 bis 3, dadurch gekennzeichnet, dass die nochmalige Veretherung bei einer Temperatur von 30 bis 100°C durchgeführt wird.

5. Verfahren nach den Ansprüchen 1 bis 4, dadurch gekennzeichnet, dass die nochmalige Veretherung bei einer Temperatur von 40 bis 70°C durchgeführt wird.

6. Verfahren nach den Ansprüchen 1 bis 5, dadurch gekennzeichnet, dass die nochmalige Veretherung bei der Temperatur durchgeführt wird, die an der Entnahmestelle des Seitenstromes herrscht.

7. Verfahren nach den Ansprüchen 1 bis 6, dadurch gekennzeichnet, das an der Destillationskolonne ein Verhältnis von Rückfluss zu Entnahme (R/E) von 0,1 bis 5 eingestellt wird.

8. Vorrichtung zur simultanen destillativen Aufarbeitung und nochmaligen Veretherung von Methyl-tert.-butylether (MTBE), i-Buten und Methanol enthaltenden Veretherungsgemischen, gekennzeichnet durch eine Destillationskolonne (5), die folgende Einrichtungen aufweist:

a) eine Einspeisungsstelle (12) für das Veretherungsgemisch,

b) am Sumpf einen an sich bekannten Umlauferhitzer (10) und eine Entnahmeeinrichtung (11) für das MTBE,

c) am Kopf einen an sich bekannten Kondensatteiler (8) für Rückfluss und Entnahme (9) des restlichen C₄-Gemisches, das frei von MTBE und weitgehend frei von i-Buten und Methanol ist,

d) eine Entnahmeleitung (6) für einen Seitenstrom oberhalb der Einspeisungsstelle (12) des Veretherungsgemisches in die Destillationskolonne (5), und

e) einen mit einem sauren Kationenaustauscher gefüllten Nachreaktor (7), in den der entnommene Seitenstrom geführt wird.

9. Vorrichtung nach Anspruch 8, dadurch gekennzeichnet, dass sie einen Zulauf (13) für den aus dem Nachreaktor (7) ablaufenden Seitenstrom im Abtriebsteil oder im Verstärkerteil bis unterhalb der Entnahmestelle für den Seitenstrom aufweist.

10. Vorrichtung nach den Ansprüchen 8 und 9, dadurch gekennzeichnet, dass sich der Zulauf (13) im Verstärkerteil bis unterhalb der Entnahmestelle für den Seitenstrom befindet.

## Claims

1. Process for the production of methyl tert.-butyl ether (MTBE) and a hydrocarbon raffinate substantially free from i-butene and methanol, from methanol and a hydrocarbon mixture containing i-butene by etherification on acid cation exchangers, characterised in that methanol and the i-butene contained in the hydrocarbon mixture are used in a molar ratio of 0.9:1 to 1:0.9, and, in the distillative separation of the etherification mixture into its constituents a side stream is taken off from the distillation column and again subjected to an etherification reaction.

2. Process according to Claim 1, characterised in that for the repeated etherification a side stream is taken off above the feed inlet point of the distillation column for the etherification mixture and passed to a secondary reactor.

3. Process according to Claims 1 and 2, characterised in that the side stream is passed in liquid phase to the repeated etherification.

4. Process according to Claims 1 to 3, characterised in that the repeated etherification is carried out at a temperature of 30 to 100° C.

5. Process according to Claims 1 to 4, characterised in that the repeated etherification is carried out at a temperature of 40 to 70° C.

6. Process according to Claims 1 to 5, characterised in that the repeated etherification is carried out at the temperature which prevails at the take-off point of the side stream.

7. Process according to Claims 1 to 6, characterised in that a ratio of reflux to take-off (R/T) of 0.1 to 5 is set at the distillation column.

8. Apparatus for the simultaneous distillative working-up and repeated etherificaiton of etherification mixtures containing methyl-tert.-butyl ether (MTBE), i-butene and methanol, characterised by a distillation column (5) which has the following devices:

(a) a feed inlet point (12) for the etherification mixture,

(b) at the bottom a reboiler (10) which is known per se and a take-off device (11) for the MTBE,

(c) at the top a condensate divider (8), which is known per se, for the reflux and take-off (9) of the remaining C₄-mixture which is free from MTBE and substantially free from i-butene and methanol,

(d) a take-off line (6) for a side stream above the feed inlet point (12) of the etherification mixture into the distillation column (5) and

(e) a secondary reactor (7) which is packed with an acid cation exchanger and into which the side stream taken off is passed.

9. Apparatus according to Claim 8, characterised in that it contains a feed inlet (13) for the side stream leaving the secondary reactor (7) in the stripping section or in the enrichment section up to below the take-off point for the side stream.

10. Apparatus according to Claims 8 and 9, characterised in that the feed inlet (13) is located in the enrichment section up to below the take-off point for the side stream.

## Revendications

1. Procédé de production d'éther de méthyle et de tertiobutyle (MTBE) et d'un raffinat hydrocarboné largement débarrassé d'isobutène et de méthanol, à partir d'un mélange d'hydrocarbures contenant du méthanol et un isobutène, par éthérification sur des échangeurs cationiques acides, caractérisé en ce qu'on utilise du méthanol et l'isobutène contenu dans le mélange d'hydrocarbures dans le rapport molaire de 0,9:1 à 1:0,9, on prélève, lors de la séparation par distillation du mélange d'éthérification en ses constituants, un courant latéral de la colonne de distillation et on soumet de nouveau ce courant à une réaction d'éthérification.

2. Procédé suivant la revendication 1, caractérisé en ce qu'on prélève un courant latéral au-dessus du point d'introduction du mélange éthérifié dans la colonne de distillation en vue de la nouvelle éthérification et on l'envoie à un postréacteur.

3. Procédé suivant les revendications 1 et 2, caractérisé en ce que le courant latéral est envoyé à la nouvelle éthérification en phase liquide.

4. Procédé suivant les revendications 1 à 3, caractérisé en ce que la nouvelle éthérification est conduite à une température de 30 à 100° C.

5. Procédé suivant les revendications 1 à 4, caractérisé en ce que la nouvelle éthérification est conduite à une température de 40 à 70°.

6. Procédé suivant les revendications 1 à 5, caractérisé en ce que la nouvelle éthérification est conduite à la température qui règne au point de prélèvement du courant latéral.

7. Procédé suivant les revendications 1 à 6, caractérisé en ce qu'on règle sur la colonne de distillation un rapport de reflux au prélèvement (R/P) de 0,1 à 5.

8. Dispositif pour le traitement simultané par distillation et l'éthérification répétée de mélanges d'éthérification contenant de l'éther de méthyle et de tertiobutyle (MTBE), de l'isobutène et du méthanol, caractérisé par une colonne de distillation (5) qui présente les aménagements suivants:

a) un point d'injection (12) pour le mélange d'éthérification,

b) à la base, un appareil de chauffage à circulation (10) connu et un dispositif de prélèvement (11) pour le MTBE,

c) à la tête, un diviseur de condensat (8) connu pour le reflux et le prélèvement (9) du mélagne en $C_4$ résiduel qui est dépourvu de MTBE et largement dépouvu d'isobutène et de méthanol,

d) un conduit (6) pour le prélèvement d'un courant latéral au-dessus du point d'injection (12) du mélange d'éthérification dans la colonne de distillation (5), et

e) un post-réacteur (7) chargé d'un échangeur cationique acide, dans lequel est envoyé le courant latéral prélevé.

9. Dispositif suivant la revendication 8, caractérisé en ce qu'il présente une admission (13) pour le courant latéral provenant du post-réacteur (7) dans la partie d'entraînement ou dans la partie de rectification jusqu'au-dessous du point de prélèvement pour le courant latéral.

10. Dispositif suivant les revendications 8 et 9, caractérisé en ce que l'admission (13) se trouve dans la partie de rectification jusqu'au-dessous du point de prélèvement du courant latéral.